# EUROPEAN PATENT APPLICATION

(11) **EP 4 837 956 A2**
(43) Date of publication of application: **11.11.2026**
(21) Application number: 25727209.6
(22) Date of filing: 26.02.2025
(51) Int. Cl.: C12N 1/06, A23L 31/15, C12N 1/16, A23J 1/18, A23J 3/20

(54) **PROCESS FOR OBTAINING A SOLUBLE SINGLE-CELL PROTEIN (PRO60) THAT CAN BE USED IN VARIOUS FOOD MATRICES**

(30) Priority: 26.02.2024 MX 2024002466
(71) Applicant: Proteo Alimentaria, S.A.P.I de C.V., Cuajimalpa (Ciudad de México), 05348 (MX)
(72) Inventor: MIGUEL ÁNGEL, Navarro Lizárraga, Colima 20020 (MX)
(74) Representative: Garrigues IP, S.L.P.
(86) International application number: PCT/IB2025/052044
(87) International publication number: WO 2025/181685

(57) **Abstract**

The present invention relates to a novel fermentation, extraction and purification process for obtaining a single-cell protein for use in the food industry, using a particular strain of *Kluyveromyces marxianus,* called KMPROTEO 1, which results in a product, consisting of a single-cell protein powder, with a protein content equal to or greater than 60%, that is highly bioavailable, which, as it is soluble, can be included in different food matrices, without changing the formulation or the profile of the product to which it is added, which we have named **"PRO60".**

The process disclosed in this specification includes the description of the aerobic fermentation of the strain *Kluyveromyces marxianus,* called KMPROTEO 1, as well as the use of the ingredient or final product obtained, by adding to some preferred food matrices.

## Description

### OBJECT OF THE INVENTION

The present invention relates to a novel fermentation, extraction and purification process for obtaining single-cell protein powder for use in the food industry, using a particular strain of *Kluyveromyces marxianus,* called KMPROTEO 1, which results in an ingredient or final product that consists of the (water-soluble) light phase derived from the cell lysis of said yeast, containing a protein concentration equal to or greater than 60% (weight/weight%).

The strain *Kluyveromyces marxianus,* called KMPROTEO 1, has been deposited under the terms of the Budapest Treaty on the International Recognition of the Deposit of Microorganisms for the Purposes of Patent Procedure, at the National Centre for Genetic Resources CNRG of the National Institute of Forestry, Agriculture and Livestock Research, where said strain, after the corresponding certification of feasibility, was assigned accession number CM-CNRG TB66.

The process disclosed in this specification includes the description of the fed-batch aerobic fermentation of the strain *Kluyveromyces marxianus,* called KMPROTEO 1, as well as the unit operations of disruption, separation and drying of the ingredient and its use as a final product, by adding to some preferred food matrices.

The present invention discloses a process for obtaining a soluble protein, which production comprises the following steps:
i) scaling the fermentation culture for biomass production of the strain *Kluyveromyces marxianus,* called KMPROTEO 1;
ii) continuously scaling the culture in a ratio of 1:10 (inoculum: total fermentation volume) wherein the amount of medium from the first fermentation process corresponds to 10% of the final volume of the following fermentation process;
iii) Fed-batch fermentation process, wherein the rate of adding nutrients is given based on the residual glucose concentration in the culture medium, which is kept below 0.5 g/l throughout the entire Fed-batch process;
iv) cell concentration by centrifugation at 5,500 - 6,500 RPM, preferably at 6,000 RPM at room temperature, and supernatant removal;
v) exposing the 10% (weight/volume) yeast biomass to autolysis at 50-60°C, preferably at 55°C;
vi) exposing concentrated yeast to enzymatic lysis using an enzyme cocktail that includes at least a cellulase, a beta-glucanase and a protease, at a temperature between 55 and 60°C; with a pH between 7 and 9, preferably at 7;
vii) exposing the yeast biomass to enzymatic lysis for 4-6 hours, preferably 5 hours;
viii)after cell lysis, carrying out centrifugation to separate the soluble phase and the heavy phase at 5,500 - 6,500 RPM, preferably at 6,000 RPM, thus subsequently separating soluble and insoluble fractions;
ix) the soluble phase, or light phase, is subjected to a microfiltration process, with a membrane having a pore size of 0.10 to 0.22, preferably 0.22 microns, to remove any heavy particles from the mixture;
x) the soluble phase is concentrated by means of an evaporator, using temperatures between 60 and 70°C, with a vacuum of -0.07 to -0.08 MPa, preferably -0.08 MPa, thus raising the solids concentration to a range between 30 and 50% (weight/volume) prior to drying;
xi) the soluble phase is concentrated by spray drying with an inlet temperature between 180 and 190°C and an outlet temperature between 80 and 90°C, thus achieving a powder with a moisture content of less than 5%.

At the end of the process described above, an ingredient is obtained that consists of a novel single-cell protein powder, with a protein content equal to or greater than 60%, that is highly bioavailable, and as it is soluble, it can be included in different food matrices, without changing the formulation or the profile of the product to which it is added, which we have named **"PRO60".**

### BACKGROUND.

Proteins are essential compounds for good human nutrition, the nutritional quality of which lies in its content of essential amino acids.

Given the exponential increase in food demand resulting from population growth, as well as the need for strategies for ethically sustainable large-scale food production, at present, the quality of a protein largely depends on the efficiency and sustainability of its production.

The search for alternative proteins that are sustainable, efficient, healthy and with a high nutritional quality, and which can organoleptically compete with plant and animal protein, represents an area of opportunity to meet market needs.

The single-cell protein (SCP), derived from microorganisms, is a viable strategy, as it uses less space and requires less time, water, energy and resources for its controlled large-scale production. The foregoing has been reported by several authors in scientific journals. An example of this is the document titled SINGLE CELL PROTEIN STATE-OF-THE-ART, INDUSTRIAL LANDSCAPE AND PATENTS 2001-2016, published by Ritala et al. in 2017, wherein a literature review of developments and patents from 2001 to 2016 concerning single-cell protein production using various substrates, processes and organisms was carried out, inferring its potential over other conventional protein sources.

Likewise, in the document titled RECENT ADVANCES IN SINGLE CELL PROTEIN USE AS A FEED INGREDIENT IN AQUACULTURE, published by Shawn W. Jones et al., the authors compared the biomass conversion efficiency and protein content of plants, animals, insects, and microorganisms such as algae, bacteria and yeasts, thus demonstrating the potential of the single-cell protein over conventional protein sources.

In the publication titled INNOVATIONS AND DEVELOPMENTS IN SINGLE CELL PROTEIN: BIBLIOMETRIC REVIEW AND PATENTS ANALYSIS, by Gislane Oliveira Ribeiro et al., the authors conducted a bibliometric analysis of publications and patents that focus on innovations and developments in single-cell protein production. In this document from 2023, the sustainable potential and increased use of this protein for food production is recognised, in addition to the importance of investing in the research and development of technologies for their production.

The single-cell protein has a good nutritional profile, depending on the microorganism used; for example, yeasts used for commercial production contain between 20% and 60% of total protein at a cellular level.

Additionally, protein extracts derived from yeasts are often a source of important vitamin groups and essential amino acids, meaning that this protein source is an appealing and innovative alternative that competes with traditional sources.

The development, discovery and creation of new high-quality protein sources for producing healthier, high quantity food is a challenge not only for the industry, but also for technological and scientific development aimed at producing protein with highly efficient processes and with an appropriate amino acid profile from yeast biomass, to thus obtain a balanced ingredient with a high concentration of nitrogen available as protein.

The single-cell protein obtained from yeast biomass is a bioavailable product obtained from conventionally consumed raw material, such as mono- and disaccharides and cheap nitrogen sources from industrial food or agri-food waste.

The production and consumption of yeast-derived protein involves the use of sustainable processes and inputs to provide the consumer with nutrients with high quality and nutritional value.

In general, single-cell protein production includes: i) the preparation of the nutrient medium for the proper and efficient growth of the yeast or microorganism involved; ii) the fermentation culture, either in solid state or in liquid state; iii) separation and concentration (in some cases, concentration only), and; iv) processing to an ingredient and food products.

The term single-cell protein, or protein from microorganisms, has two connotations. The first connotation refers to the use of the whole cell that contains the desired protein within the boundaries of the cell wall, which is less available or functional; the second connotation refers to the protein that has been isolated or separated from the cellular entity of the microorganism, which has better quality and a higher concentration. In the latter scenario, degradation of the cell wall, if necessary for the characteristics of the final ingredient, is carried out to make the intracellular protein of the yeasts more accessible (or bioavailable).

Traditionally, several methods have been used for yeast cell disruption, which include: i) mechanical force; ii) chemical disruption with detergents; enzymatic disruption, and; iii) autolytic disruption, from the induction of cell autolysis, or a combination of the previous methods.

Cell autolysis has been used for the commercial production of single-cell proteins, such as Marmite^{®} and Vegemite^{®} products, which are edible spreads made from yeast extract; induction is carried out by heating the yeast cells to between 45-50°C for sufficient time for intracellular enzymes to partially hydrolyse the cell wall. It is worth mentioning that in this process the proteins are also reduced to small peptides, altering the quality and quantity of the original protein.

Several processes for extracting yeast cell protein have already been patented. An example of this is European Patent EP3670646A1, called Functional Yeast Protein Concentrate, which discloses a method for performing mechanical lysis at pH 6.5-8.5 to extract protein from a cell suspension of the yeast *S*. *cerevisiae.* Cell lysis is carried out at a temperature below 40°C; wherein the soluble fraction is filtered to reduce the content of molecules smaller than 30 kDa; and said fraction is dried. The protein concentrate, which has a discreet flavour, comprises a large number of folded proteins, so they are able to aggregate to form a solid protein matrix when heated. These characteristics allow the product to be added to various food matrices.

In the same manner, several methods have been proposed to improve the nonmechanical lysis of yeast creams, which commonly involve the autolytic ability of said microorganisms to produce enzymes that degrade the cell wall itself. Such is the case of US patent US4810509A, called METHOD FOR PRODUCING YEAST EXTRACT (KANEGAE Yukihiro, SUGIYAMA Yoshio, MINAMI Kanshiro), wherein the tasty yeast extract is obtained by heating a biomass suspension to 55°-70°C, allowing the cells to rupture at a pH between 8 and 10.

Some more current methods have proposed the use of agents that help to improve the autolytic process, such as chitosan from Japanese patent EP0466922A1, called PRODUCTION of yeast extract (ORIGANE, Akira; SATO, Takasi), in which the autolytic method for *S*. *cerevisiae* and *P. pastoris* was improved by adding 0.01-3% weight/weight chitosan, at a pH of 2.5-7.5, at 30-54°C for 10-20 hours.

Another similar case involves adding enzymes that assist in the hydrolysis of carbohydrates and proteins in the cell wall of yeasts. This is discussed in US patent US11602156B2, called YEAST PROTEINS (Rudy Menin, Pauline Spolaore, and Isabelle Mouly; 2018), which provides a method in which a yeast cream is exposed to thermal plasmolysis at a temperature between 70 and 95°C, for a period between 30 seconds and 4 hours; subsequently, the insoluble fraction and the soluble fraction are separated, to finally subject the insoluble fraction to the activity of at least one ribonuclease enzyme and a glucanase, sequentially or simultaneously, at a temperature between 40 and 65°C, for a period of 8 to 24 hours. Furthermore, in said patent, the insoluble fraction, which has no flavour, has a nucleotide content of less than 3% and a protein content of at least 72%, which ensures its quality and safety for human consumption. (This patent corresponds to application US17049897).

Food safety is a very important issue as it is aimed at protecting the welfare of the consumer of the final product. To this effect, there are various regulatory bodies that determine whether an ingredient or product poses health risks, such as the Food Agriculture Organization (FAO) of the United Nations and the Food and Drug Administration (FDA), as well as the different country-specific governmental bodies.

In the case of the single-cell protein, an important step in ensuring its consumption is the removal of nucleic acids, as the rapid production of biomass involves a high content of nucleic acids: cellular DNA and RNA; said content depends on the conditions of cell growth, such as nutrients in the fermentation culture or even the Carbon/Nitrogen ratio.

Specifically, high concentrations of nucleic acids and purines represent a problem for human health, as they increase the concentration of uric acid in the blood plasma and thus lead to the occurrence of kidney stones and the suffering of gout.

In products available on the market, some methods to lower the concentration of purines in yeast-derived products involve an increase in temperature (60°C-100°C) to induce nucleic acid degradation by endogenous ribonucleases or the use of immobilised exogenous ribonucleases. The increase in temperature also induces the diffusion of degraded DNA and RNA to the cell exterior, meaning that the cost of this process lies in the increase in temperature.

Currently most of the state of the art, which presents a solution to this problem, uses changes in temperature to induce nucleotide lysis, as in the case of US patent US3947605A, called PROCESS FOR PREPARING HIGH YIELDS OF SINGLE CELL PRODUCTS HAVING REDUCED PURINE CONTENT AND A HIGH NUTRITIVE VALUE (CHAO, Kwei C). Other patents propose pH changes in an aqueous medium to carry out cell lysis or to precipitate proteins from the protein fraction, as is the case with US patents: US3887431, called YEAST PROTEIN ISOLATE WITH REDUCED NUCLEIC ACID CONTENT AND PROCESS OF MAKING SAME (Ernest Aleck Robbins et al.); and patent US3991215A called MANUFACTURE OF PROTEIN ISOLATE HAVING A REDUCED NUCLEIC ACID CONTENT BY A THERMAL PROCESS (Ernest Aleck Robbins).

The strains belonging to the species of *Kluyveromyces marxianus* have been isolated from a wide variety of habitats (fruits, rotten sisal leaves, wastewater from a sugar factory, "Lufthefe", etc.). The first time this species was described was in 1888 by E. C. Hansen, after being isolated and named *Saccharomyces marxianus.* The genus was first described as *Kluyveromyces* in 1956 and was divided into 6 clades in 1998.

*Kluyveromyces marxianus* is a highly diverse species, metabolically and polymorphically speaking, which has characteristics that are relevant to the food industry from a sustainability and biotechnological point of view. Among its characteristics are its high growth rates due to its small size, which allows it to grow faster than other organisms; it has a wide range of carbohydrate intake, such as residual whey lactose, molasses and inulin; its ability to produce compounds of industrial interest, such as enzymes for the hydrolysis of carbohydrates and proteins; as well as its ability to glycosylate therapeutic proteins, or its ability to produce ethanol at a temperature of 40°C-45°C.

Some strains have been shown to have protein secretion and biomass production capacity, resulting in their use for producing enzyme preparations and their possible use for expressing recombinant proteins, such as the strain *Kluyveromyces marxianus* CGMCC10621, described in Chinese patent CN105087403Acalled KLUYVEROMYCES MARXIANUS AND ITS APPLICATIONS (LYU HONG, et al.).

Another important metabolic characteristic is that, unlike yeasts of the species *Saccharomyces, Kluyveromyces marxianus* have a strong *"negative Crabtree"* effect, as no ethanol is produced after a glucose pulse is applied to the cells during aerobic growth. In other words, when oxido-reductive metabolism is activated in *Kluyveromyces marxianus* as a function of increased glycolytic flux, the maximum reproductive capacity of the cells is not achieved.

Some studies have demonstrated the potential of *Kluyveromyces marxianus* biomass as an excellent alternative to replace *Saccharomyces cerevisiae* for the production of yeast autolysates, insoluble glucans at alkaline pH, glucans and natural bio-emulsifiers.

Paul J. Anderson, in his scientific publication titled THERMOTOLERANT SINGLE CELL PROTEIN PRODUCTION BY KLUYVEROMYCES MARXIANUS VAR. MARXIANUS*,* and Gisela Paez, in her technical publication titled "Continue Production of Single CELL PROTEIN OF KLUYVEROMYCES MARXIANUS VAR. MARXIANUS FROM DILUTED CHEESE WHEY", showed that various strains of *Kluyveromyces marxianus* grown in molasses have a good amino acid profile for single-cell protein production, with a balanced distribution compared to other yeasts and international reference standards (FAO), as well as with conventional protein sources such as egg and wheat. The foregoing suggests the potential use of this single-cell protein. Moreover, as described in Chinese patent CN105707436A called APPLICATIONS OF *KLUYVEROMYCES YEAST* (LYU HONG; MO WENJUAN; ZHOU JUNGANG; YU YAO), some strains of *Kluyveromyces marxianus* have already been applied as safe animal feed additives wherein they furthermore improve digestion.

The application WO2011140649A1 called Fermentation process of a substrate using a mixed culture for the production of an edible biomass for animal and/or human consumption (LEWANDOWSKI, Raymond [CA]), relates to a process comprising a combination of yeasts and bacterial strains for the production of consumable biomass from substrates comprising a simple sugar. More particularly, the claimed subject matter includes the use of *Lactobacillus fermentum, Kluyveromyces marxianus* and *Saccharomyces unisporus.*

Many strains of *Kluyveromyces marxianus* have already been accepted as GRAS, as well as other strains commonly used in the food industry, such as *Saccharomyces cerevisiae* and *Kluyveromyces lactis.* Compared to its predecessors, *Kluyveromyces marxianus* has better characteristics of industrial interest, including without genetic improvement (GMO-free). These characteristics include its natural ability to produce extracellular enzymes, such as pectinases and cellulases, which facilitate and reduce the cost of their concentration and separation processes for release during protein production, being less viscous than their fungal counterparts.

*Kluyveromyces marxianus* has turned out to be an emerging, non-pathogenic yeast, with great potential for use in the food and biomedical industries, particularly for single-cell protein production or recombinant protein expression; however, there is little information on biomass scaling systems for the same at the industrial level.

Some patents disclose Batch or Fed-batch systems for *Kluyveromyces marxianus* biomass production. Chinese patent WO2017036294A1 called KLUYVEROMYCES MARXIANUS AND ITS APPLICATIONS (LV HONG et al.) discloses a Fed-batch system to produce biomass from the strain *Kluyveromyces marxianus* (with deposit number CGMCC 10621), a hyper-secretory strain of recombinant proteins, in which its growth from 50 g/l glucose and 40 g/l yeast extract, at a temperature between 28 and 35°C; pH of 7.0, 30% DO, is proposed, wherein during the Fed-batch process a residual glucose concentration in the medium between 5 and 15 g/l is kept constant.

In the state of the art, fermentation methods from cheap nutrient sources (derived from the food industry) have been proposed for *Kluyveromyces marxianus var. lactis* biomass production, such as patent application BG48706A1 called METHOD FOR PREPARING PROTEIN (SIMOVA, Emilina et al.), which proposes to use deproteinised, ultrafiltrated and improved whey from the addition of 0.5-0.9% (NH4)2HPO4 and 10-20% maize extract, at a pH of 5.0, to culture the strain *Kluyveromyces marxianus var. lactis* NBPMCC1502 for 24 hours with aeration, at a rate of 0.008 s⁻¹, at 52.3 rad/s and a temperature of 28 to 32°C.

The processes available in the state of the art do not synergistically resolve the consecutive process of intracellular protein release and the consecutive steps for generating products for the food industry.

The strain *Kluyveromyces marxianus,* called KMPROTEO 1, protected under Mexican patent MX 402824 B, has an increased capacity for obtaining single-cell biomass and subsequently single-cell protein from the fermentation of whey. The fermentation process of this patent uses cheese whey as a propagation medium and allows a product with a high protein value to be obtained which will be in the range of 50 to 90%, the strain being KMPROTEO 1 in particular, which is used in the novel process of the invention disclosed in terms of this specification.

### BRIEF EXPLANATION OF THE FIGURES.

The novel aspects that are considered to be characteristic of the fermentation, extraction and purification process for obtaining a single-cell protein for use in the food industry, using a particular strain of *Kluyveromyces marxianus,* called KMPROTEO 1, disclosed in this document, will be set out in particular in the accompanying claims; however, the invention itself, both in terms of its structural organisation and in conjunction with other objects and advantages thereof, will be better understood in the following detailed description of certain preferred embodiments when read together with the accompanying figures, wherein:
Figure 1 shows the flow diagram of the process for producing a soluble single-cell protein disclosed in the present invention.
Figure 2 shows the macroscopic morphological characterisation of the strain *Kluyveromyces marxianus* KMPROTEO 1, on PDA solid medium at 24 and 96 hours of growth, at 35°C, under aerobic conditions.
Figure 3 shows the microscopic morphology of *Kluyveromyces marxianus* KMPROTEO 1, after being grown on a PDA solid medium, at 24 and 96 hours, at 35°C under aerobic conditions.
Figure 4 shows the exponential growth kinetics of the yeast *Kluyveromyces marxianus* KMPROTEO 1, in the medium C (described below) during the Fed-batch fermentation process, under the conditions mentioned in this specification.
Figure 5 is a graph that shows the exponential growth phase of the Fed-batch fermentation process in natural logarithm (Ln) over time. The slope was used to calculate the specific growth rate of the yeast *Kluyveromyces marxianus* KMPROTEO 1 in the medium C during the Fed-batch fermentation process, under the conditions mentioned in this document.

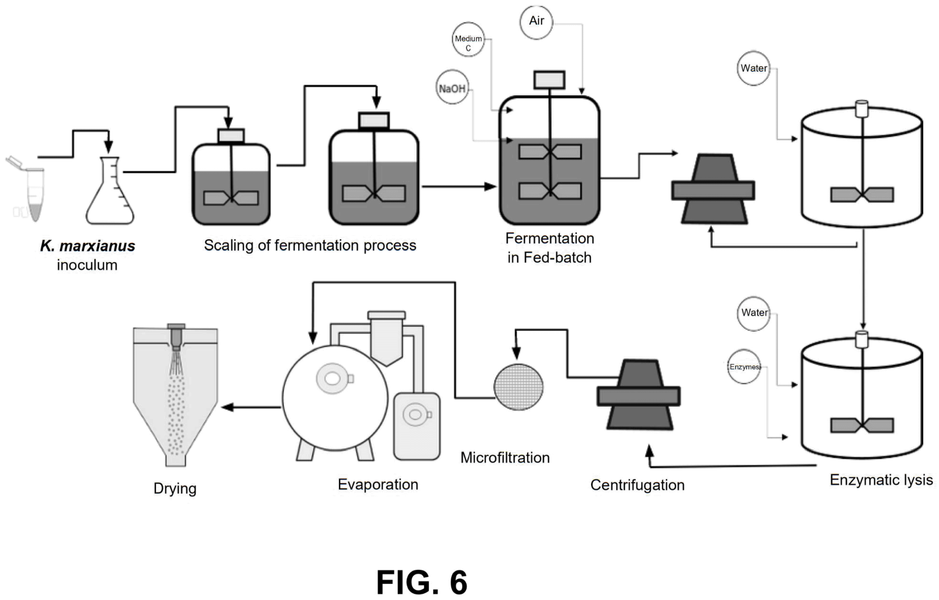

Figure 6 shows the diagram of the process for producing the soluble single-cell protein disclosed in the present invention, showing the relevant equipment per unit operation.

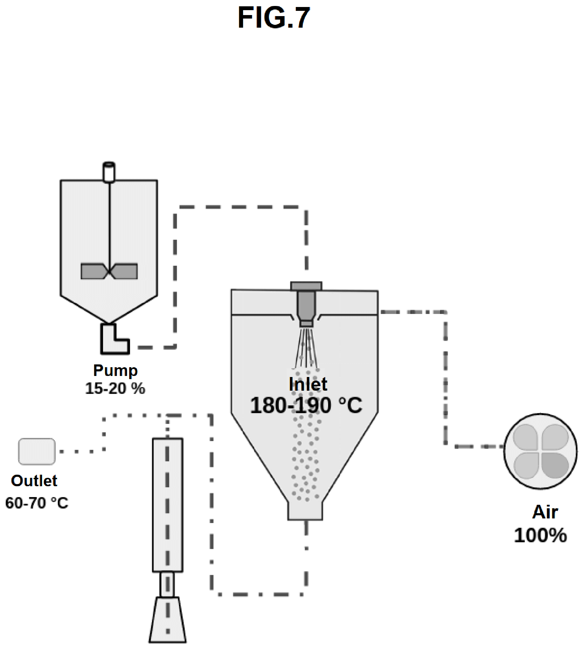

Figure 7 shows the drying process for producing for the soluble single-cell protein disclosed in the present invention, which results in a soluble powder.

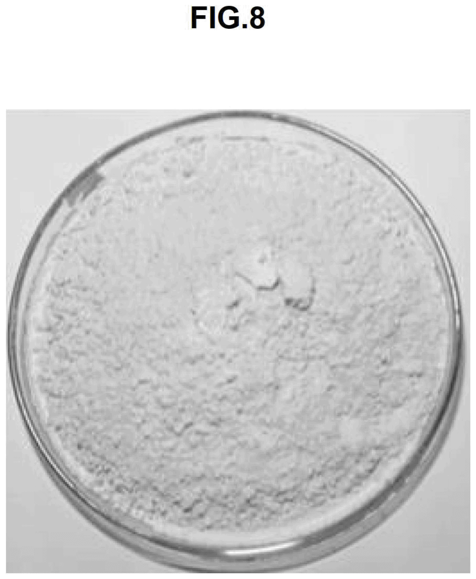

Figure 8 shows the powder product, the result of the process for producing a single-cell protein from *Kluyveromyces marxianus* KMPROTEO 1.

### DETAILED DESCRIPTION OF THE INVENTION.

This specification discloses the process for obtaining an ingredient that consists of a novel single-cell protein powder, with a protein content equal to or greater than 60%, that is highly bioavailable, and as it is soluble, it can be included in different food matrices, without changing the formulation or the profile of the product to which it is added, which we have named **"PRO60".**

The disclosed process involves the standardisation of four steps after fermentation and biomass washing: i) cell disruption; ii) separation of soluble and insoluble cell phase; iii) soluble phase concentration, and; iv) soluble phase drying.

The product resulting from the novel process disclosed in this specification is a food grade additive, very rich in protein, amino acids and oligopeptides, as well as in some organic acids, nucleic acids and mineral salts (to a lesser extent).

The powder product resulting from the novel process disclosed in this specification has an acidic to neutral flavour and a pleasant characteristic odour. Given its organoleptic, functional and nutritional attributes, the product resulting from the process disclosed in this specification can be added to various food matrices, such as sausages, ice creams, pastas, sauces, breads and fried foods, by adding nutritional properties to these matrices.

In the food industry, protein extracts, such as those disclosed in the present invention, are often added to improve organoleptic characteristics, such as flavour, odour and texture to various conventional products, adding high nutritional value to them compared to other food additives.

In the table below, the main characteristics of the single-cell protein of the present invention are shown.

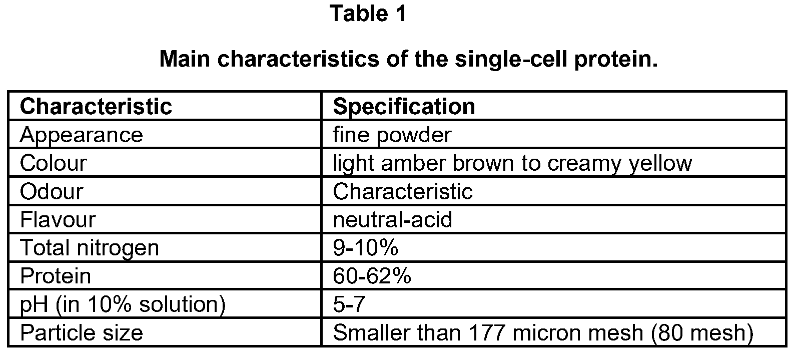

The final product, a result of the process disclosed in this specification, is functionally stable and safe for consumption.

The good yield of biomass production and extraction of the highly available, soluble protein-rich phase allows the production of the product to be scalable to industrial volumes in an easy and replicable way.

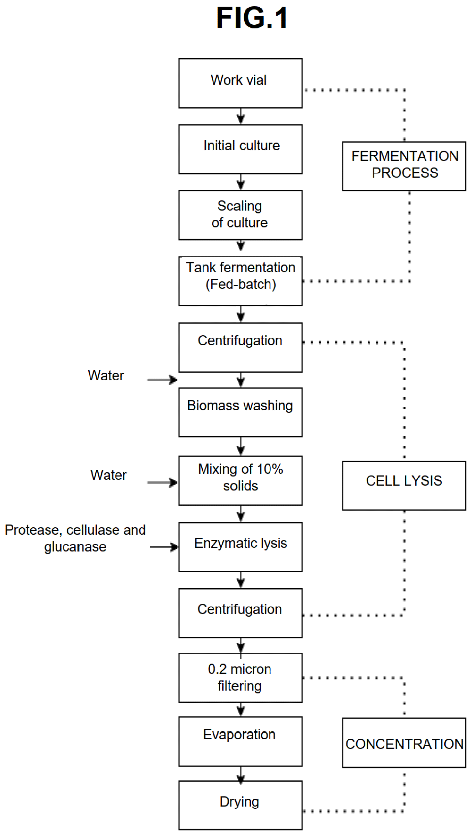

The process for producing the single-cell protein powder, disclosed in this specification, the flow diagram of which is shown in Figure 1, has production and scalability advantages over other protein sources for the food industry.

The yeast strain *Kluyveromyces marxianus* KMPROTEO 1, used in the process disclosed in this specification, is a eukaryotic microorganism, with a high cell growth rate in a dextrose-rich medium, as the main source of carbon, and yeast extract and ammonium sulphate as the main source of nitrogen. Said strain belongs to the kingdom Fungi, phylum *Ascomycota,* subphylum *Saccharomycotina,* class *Saccharomycetes,* order *Saccharomycetales,* family *Saccharomycetaceae,* genus *Kluyveromyces* and species *Kluyveromyces marxianus.*

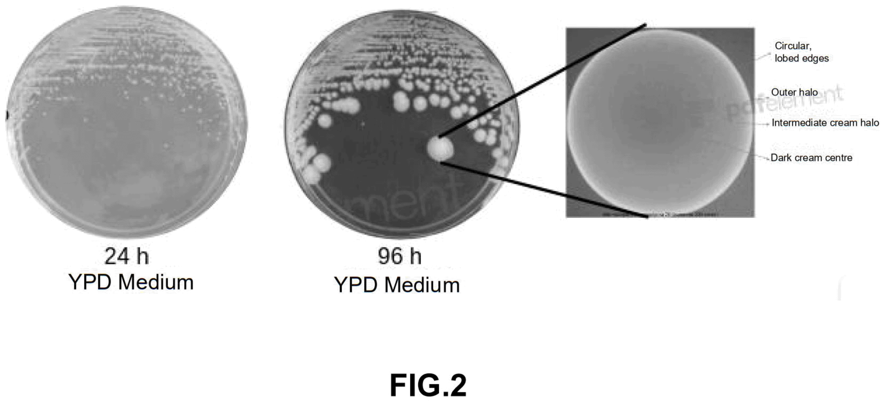

The strain KMPROTEO 1 has a spherical ovoid, creamy, white colonial morphology in YPD (yeast extract, peptone and dextrose) medium. At the microscopic level, it is about 1.9-2.5 µm wide and 2.8-4 µm long, and reproduces by polar budding and has ascospore formation, as shown in Figure 2.

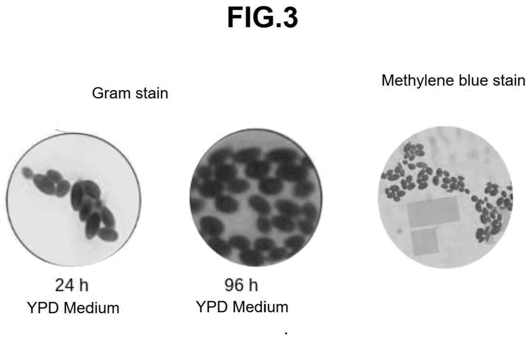

The strain KMPROTEO 1, used in the process disclosed in this specification, was selected for its high growth rate of µ = 0.1/h under the conditions described herein, at a temperature of 35°C and 200 rpm; its biomass production corresponds to 60 g/l/h and a protein content in the finished product of more than 50%, as shown in Figure 3.

Next, the preferred fermentation processes of cell disruption, concentration and drying will be described.

### 1. FERMENTATION PROCESS

This specification firstly discloses an optimised process for producing yeast biomass on a commercial scale, from a culture fed under aerobic conditions, the preferred fermentation time of which is 48-96 hours, to reach a cell concentration of 60 g/l biomass, using the strain *Kluyveromyces marxianus* KMPROTEO 1. Said fermentation process for producing single-cell protein consists of the following steps:
i) Yeast reactivation: To reactivate the strain KMPROTEO 1, 200 microlitres of the cryopreserved yeast (concentration of 1x10⁸⁻¹⁰ CFU/ml) are inoculated in a flask with a nominal volume of 1 L ml, which contains 400 ml of medium A (described in Table 2 below). The inoculated flasks are incubated at 35°C for 12 hours, at a stirring rate of 300 RPM. 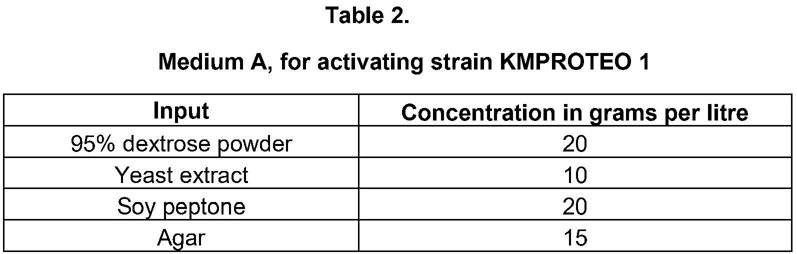
ii) Scaling: After the incubation time, the 400 ml from the previous step are inoculated in 3.6 L of medium B (described in Table 3 below) in a stirred tank fermenter to form a total volume of 4 litres, wherein the concentration of the inoculum corresponds to 10% (volume/volume) of the culture. The inoculated reactor is placed at an air flow of 1 vvm (volume of aeration per volume of medium per minute), at a propeller stirring rate of 200 RPM, at a fermentation temperature of 35°C ± 2°C; pH of between 5 and 5.5. The culture is fermented for 4 to 6 hours to an optical density (OD 600 nm) greater than 1.

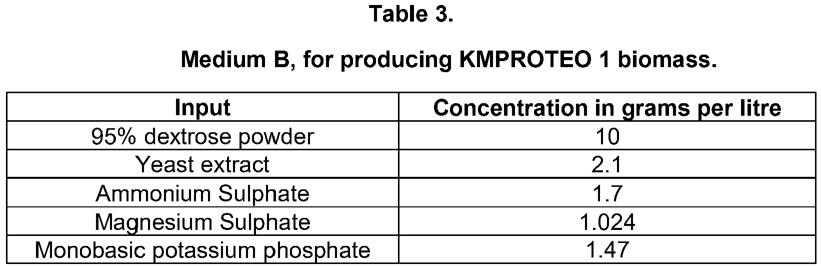

The 4 litres obtained from the previous culture are inoculated into 36 litres of medium B (Table 3), in a nominal 100 litre stirred tank bioreactor, maintaining the same media ratio as described above, giving a total of 40 L of work. The fermentation conditions remain the same as in the previous step.

After the fermentation time, the 40 litres are pumped into the production reactor, which must have a nominal capacity of 1,000 litres, wherein the Batch fermentation (closed batch) starts with a volume of 360 litres. For the Batch process, the same operating parameters as mentioned above, as well as the same concentration of the previous culture medium, are maintained.

Once 400 litres of fermentation in Batch mode are obtained at an optical density of >1.0 (OD 600 nm), the Fed-Batch fermentation starts, wherein the continuous addition of nutrients found in the medium C (described in table 4 below) is carried out; at the end of the process, 200 litres of medium C (Table 4) will have been added to complete a total volume of 600 litres at a concentration of 60 grams per litre and a total biomass of 36 kg.

The addition of medium starts when the pH of the previous step starts to drop and reaches 3; the optical density of the culture is greater than or equal to 10 OD; and the biomass concentration is greater than 4 grams per litre.

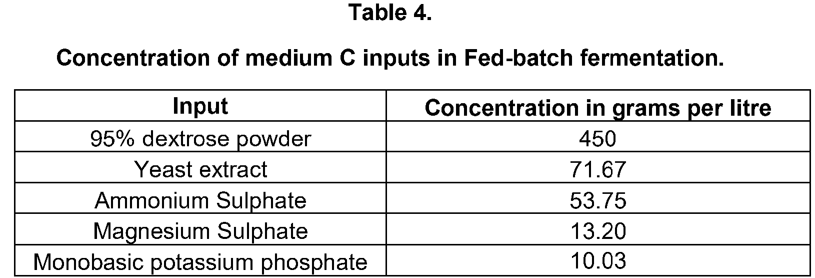

During the Fed-batch fermentation model, the following parameters are maintained in the operation (µ = 0.10 h-1 depending on the volume):
a) The continuous addition of medium C (Table 4) is carried out in such a way that the concentration of residual glucose in the fermentation medium is always less than 0.5 g/l and the concentration of ethyl alcohol is always less than 1 g/l
b) A pH in the range 5 to 5.5 is maintained by constant addition of 1 N sterile sodium hydroxide
c) Oxygenation of the culture must be constant and greater than 50%
d) The concentration of foam must be controlled by the addition of previously prepared and sterilised defoamer at a concentration of 70% (volume/volume)
e) The respiratory quotient (RQ) of fermentation between 1 and 1.5

The culture is released when 60 grams per litre of biomass is achieved in the production reactor.

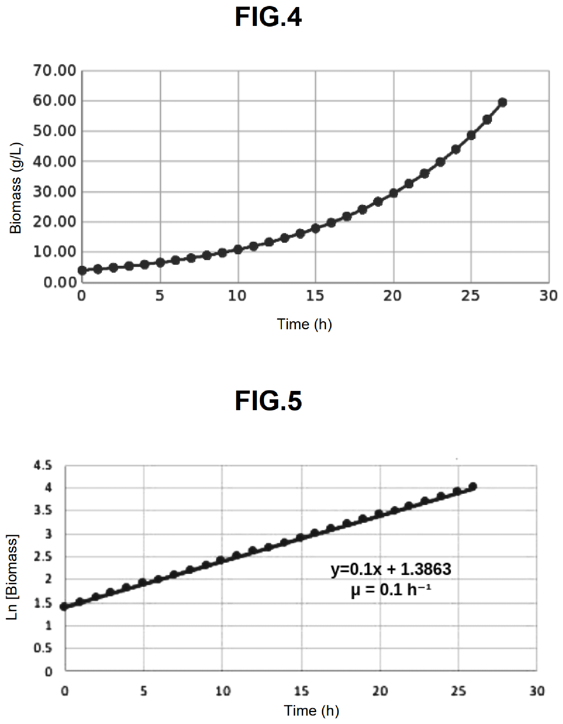

In Figure 4, the exponential growth kinetics of the yeast *Kluyveromyces marxianus* KMPROTEO 1 is shown in grams per litre (g/l) over time in hours (h), in medium C (Table 4) during the Fed-batch fermentation process, under the conditions mentioned in this document, wherein the maximum biomass production peak is given between 25 and 30 hours, this being 60 g/l.

Figure 5 is the graph that shows the exponential growth phase of the Fed-batch fermentation process in natural logarithm (Ln) over time. The slope was used to calculate the specific growth rate of the yeast *Kluyveromyces marxianus* KMPROTEO 1 in medium C (Table 4) during the Fed-batch fermentation process, under the conditions described in this document.

### 2. CELL DISRUPTION

Once the required cell concentration has been obtained, the culture is pumped into a buffer tank, which feeds a rotating disc centrifuge, which is required to concentrate the cell biomass, separating it from the rest of the supernatant. The biomass is centrifuged at a rate of 5,500 - 6,500 RPM, preferably 6,000 RPM, with discharges lasting 4 minutes; opening of 1s; and pumping of 360L/h.

The biomass is re-suspended in an equal volume of process water and recentrifuged under the same conditions to remove residual compounds from the culture medium.

Lastly, the biomass is dissolved in water to obtain a 10% (weight/volume) solution.

After concentrating the biomass by centrifugation, it is re-suspended in water, bringing it to a solids percentage between 10% and 20% (weight/volume). Subsequently, the mixture is pumped into an enzyme reactor, wherein enzymatic cell lysis is carried out, using an enzyme cocktail, which includes cellulases, beta-glucanases and proteases, at a percentage (weight/weight) of 1.0, 1.0 and 0.2% respectively, with the following control parameters: temperature between 55°C and 60°C, preferably 55°C; with a pH between 7 and 9, and; with a hydrolysis time between 4 and 6 hours. Said enzymes allow cell lysis and protein release to be improved under the same conditions as cell autolysis.

For the intracellular protein release process of the strain *Kluyveromyces marxianus* KMPROTEO 1, a disruptive cell lysis method is used, consisting of two foundations: i) autolysis, and; ii) enzymatic lysis. Said method is standardised based on the conditions previously described in the literature.

The disruption obtained by enzymatic hydrolysis coupled to cell autolysis, under the aforementioned conditions, are the optimum method for the process disclosed in this specification, because it releases a significant amount of protein into the soluble medium, in a range between 25% and 30% solubilised solids to the medium, with a protein concentration between 60% and 62%, as well as a low concentration of nucleic acids generated during fermentation.

The suspended biomass was placed under different conditions, as described in the following Table:

**Table 5.**

| **Effect of TEMPERATURE variation, and pH in the cell disruption of *K. marxianus* KMPROTEO 1.** | | | | |
|---|---|---|---|---|
| **Type of lysis** | **pH** | **Temperature** | **Time** | **Conditions** |
| **Autolysis** | 5.5 and 8.13 | 55°C | 6 h | - , 130 rpm |
| **Mechanical** | 5.5 | 55°C | Two passages | 7,000 psi, 130 rpm |
| **Enzymatic** | 5.5 | 55°C | 6h | 1% cellulase 25K and 0.2% protease |

| | | | | |
|---|---|---|---|---|
| VARIABLES: temperature of 55°C; pH of 5.6 and 8; lysis time of 6 h. DISRUPTION METHOD: enzymatic lysis, autolysis; mechanical lysis at 7,000 psi (two passages). | | | | |

For intracellular protein release, the disruption by enzymatic lysis disclosed in this specification is a novel method, as it releases a significant amount of protein into the soluble medium, in a range between 28% and 33% solubilised solids to the medium, thus achieving a high protein concentration in the final product powder, in the range of 60-63%, while reducing the content of nucleic acids generated during fermentation.

### 3. CONCENTRATION

After hydrolysis, the mixture is returned to the feed tank of the centrifuge and centrifuged, thus separating the product into 2 flows and obtaining a soluble (or light) phase and an insoluble (or heavy) phase, thus separating soluble and insoluble fractions. Centrifugation is carried out at a rate of 5,500 - 6,500 RPM, preferably 6,000 RPM, with discharges lasting 4 minutes; opening of 1 s; with pumping of 360 L/h.

Consecutively, the soluble phase is subjected to microfiltration using a ceramic filter with a pore size between 0.10 and 0.22, preferably 0.22 microns at a filtration rate of 20 MI/min (10-30% pump power), a transmembrane pressure of less than 0.5 bar, and a constant pumping pressure between 0.25 and 0.30 bar, thus making the product free of insoluble particles larger than 0.22 micrometres. The permeate product passes to a balance tank prior to the next unit operation. During this process, a loss of 2-10% of total protein is obtained.

The soluble phase is concentrated by means of an evaporator at a temperature between 50°C and 70°C (preferably at 60°C) and a vacuum between -0.07 and -0.08 MPa, preferably -0.08 and -0.1 Kpa, thus evaporating water from the extract and therefore raising the solids concentration of the mixture to a range between 30 and 50% (weight/volume) prior to drying.

### 4. DRYING

Once the protein extract has been concentrated to 30%-50% solids (weight of solids/volume of water), the drying process is started (preferably at 30%), wherein the soluble phase is spray dried at an inlet temperature between 180°C and 190°C and an outlet temperature between 70°C and 90°C (preferably at 80°C), at a constant flow rate of 8.5 L/h, at a solids concentration of 30%; and an atomisation pressure of 5 Kgf/cm². Atomiser rate: 550 Hertz- 30,000 RPM. Air flow rate: 13 m/s.

The product obtained will be a fine powder, with a light amber brown/creamy yellow colour with a moisture content equal to or less than 5%.

The powder ingredient obtained from the novel process disclosed in this specification is added to various food matrices, such as cheeses, yoghurt or spreadable dips, achieving homogeneous incorporation and a finished product with superior sensory qualities, while maintaining a vegan profile.

### CHARACTERISTICS AFTER DRYING:

- Light amber brown to creamy yellow colour
- Appearance of fine powder
- Particle size passed >99% in mesh # 60
- Total N: 9-10%
- Protein close to >60%, between 60 and 62%

### INDUSTRIAL APPLICABILITY.

The novel fermentation, extraction and purification process for obtaining a single-cell protein for use in the food industry, using the strain *Kluyveromyces marxianus,* called KMPROTEO 1, derived from the cell lysis of said yeast, containing a protein concentration equal to or greater than 60% (weight/weight%) and the product directly obtained from the process, which results in a water-soluble ingredient or final product, are concepts that clearly meet the requirement of industrial applicability.

It is noted that, with regard to the present date, the best method known to the applicant for carrying out the aforementioned invention is the one that is clear from reading this description.

Although the invention has been described in some detail, by way of illustration and examples for clarity of understanding, it will quickly become apparent to those with normal experience in the art that, in the light of the teachings of this invention, changes and modifications can be made to it, without departing from the spirit or scope of the description, including the attached claims.

## Claims

1. A fermentation, extraction and purification process for obtaining a single-cell protein with a protein content equal to or greater than 60% for use in the food industry, wherein a particular strain of the yeast *Kluyveromyces marxianus,* called KMPROTEO 1, deposited under the terms of the Budapest Treaty, with accession number CM-CNRG TB66, is used in said process.

2. The process according to claim 1, which results in obtaining a soluble single-cell protein, wherein said process comprises the following steps:
i) scaling the fermentation culture for biomass production of the strain *Kluyveromyces marxianus,* called KMPROTEO 1;
ii) continuously scaling the culture in a ratio of 1:10 (inoculum: total fermentation volume) wherein the amount of medium from the first fermentation process corresponds to 10% of the final volume of the following fermentation process;
iii) Fed-batch fermentation process, wherein the rate of adding nutrients is given based on the residual glucose concentration in the culture medium, which is kept below 0.5 g/l throughout the entire Fed-batch process;
iv) cell concentration by centrifugation at 6000 RPM at room temperature, and supernatant removal;
v) exposing the 10% (weight/volume) yeast biomass to autolysis at a temperature between 50°C-60°C, preferably at 55°C;
vi) exposing concentrated yeast to enzymatic lysis using an enzyme cocktail that includes at least a cellulase, a beta-glucanase and a protease, at a temperature between 55°C and 60°C; with a pH between 7 and 9 (preferably 7);
vii) exposing the yeast biomass to enzymatic lysis for 4-6 hours, preferably 5 hours;
viii)after cell lysis, carrying out centrifugation to separate the soluble phase and the heavy phase at 6,000 RPM, thus subsequently separating soluble and insoluble fractions;
ix) the soluble phase, or light phase, is subjected to a microfiltration process, with a membrane having a pore size of 0.10 to 0.22, preferably 0.22 microns, to remove any heavy particles from the mixture;
x) the soluble phase is concentrated by means of an evaporator, using temperatures between 60°C and 70°C, with a vacuum of -0.08 MPa, thus raising the solids concentration to a range between 30 and 50% (weight/volume) prior to drying;
xi) the soluble phase is concentrated by spray drying with an inlet temperature between 180°C and 190°C and an outlet temperature between 80°C and 90°C, thus achieving a powder with a moisture content of less than 5%.

3. A method for scaling the biomass of the strain *Kluyveromyces marxianus* KMPROTEO 1, described in claim 2, wherein the fermentation culture consists of the following steps:
(i) yeast reactivation: To reactivate the strain KMPROTEO 1, by inoculation of 200 microlitres of the cryopreserved yeast (concentration of 1x10⁸⁻¹⁰ CFU/ml), in a flask with a nominal volume of 1 L ml that contains 400 ml of the medium "A" (described in Table 2 below), and subsequent incubation of the inoculated flasks at 35°C for 12 hours at a stirring rate of 300 RPM;
**Table 2**
| **Medium A, for activating strain KMPROTEO 1** | |
|---|---|
| **Input** | **Concentration in grams per litre** |
| 95% dextrose powder | 20 |
| Yeast extract | 10 |
| Soy peptone | 20 |
| Agar | 15 |
(ii) scaling comprising the following steps:
• after the incubation time, the 400 ml from the previous step are inoculated in 3.6 L of medium "B" (described in Table 3, below) in a stirred tank fermenter, to form a total volume of 4 litres, wherein the concentration of the inoculum corresponds to 10% (volume/volume) of the culture, wherein the inoculated reactor is placed at an air flow of 1 vvm (volume of aeration per volume of medium per minute), a propeller stirring rate of 200 RPM, at a fermentation temperature of 35°C ± 2°C; pH between 5 and 5.5 and wherein the culture is fermented for 4 to 6 hours, to an optical density (OD 600 nm) greater than 1;
**Table 3**
| **Medium B, for producing KMPROTEO 1 biomass.** | |
|---|---|
| **Input** | **Concentration in grams per litre** |
| 95% dextrose powder | 10 |
| Yeast extract | 2.1 |
| Ammonium Sulphate | 1.7 |
| Magnesium Sulphate | 1.024 |
| Monobasic potassium phosphate | 1.47 |
• the 4 litres obtained from the previous culture are inoculated into 36 litres of medium B (Table 3), in a nominal 100 litre stirred tank bioreactor, maintaining the same media ratio as described above, giving a total of 40 L of work, keeping the fermentation conditions the same as in the previous step;
• after the fermentation time, the 40 litres are pumped into the production reactor, which must have a nominal capacity of 1,000 litres, wherein the Batch fermentation (closed batch) starts with a volume of 360 litres, maintaining the same operating parameters as mentioned above, as well as the same concentration of the previous culture medium;
• once 400 litres of fermentation in Batch mode are obtained at an optical density of >1.0 (OD 600 nm), the Fed-Batch fermentation starts, wherein the continuous addition of nutrients found in the medium C (described in Table 4 below) is carried out; at the end of the process, 200 litres of medium "C" will have been added to complete a total volume of 600 litres at a concentration of 60 grams per litre and a total biomass of 36 kg, wherein the addition of medium starts when the pH of the previous step starts to drop and reaches 3, the optical density of the culture is greater than or equal to 10 OD, and the biomass concentration is greater than 4 grams per litre, according to the concentration of inputs described in the table below:
**Table 4**
| **Concentration of medium C inputs in Fed-batch fermentation.** | |
|---|---|
| **Input** | **Concentration in grams per litre** |
| 95% dextrose powder | 450 |
| Yeast extract | 71.67 |
| Ammonium Sulphate | 53.75 |
| Magnesium Sulphate | 13.20 |
| Monobasic potassium phosphate | 10.03 |
wherein during the Fed-batch fermentation model, the following parameters are maintained in the operation (µ = 0.10 h-1 depending on the volume):
a) the continuous addition of medium "C" (Table 4) is carried out in such a way that the concentration of residual glucose in the fermentation medium is always less than 0.5 g/l and the concentration of ethyl alcohol is always less than 1 g/l;
b) a pH in the range 5 to 5.5 is maintained by constant addition of 1 N sterile sodium hydroxide;
c) oxygenation of the culture must be constant and greater than 50%;
d) the concentration of foam must be controlled by the addition of previously prepared and sterilised defoamer at a concentration of 70% (volume/volume);
e) the respiratory quotient (RQ) of fermentation between 1 and 1.5;
and wherein the culture is released when 60 grams per litre of biomass is achieved in the production reactor.

4. A method for fermenting the strain *Kluyveromyces marxianus* called KMPROTEO 1, described in claim 2, is performed in Fed-batch, which includes kinetic and control parameters, optimised for single-cell protein production at any scale with the strain *Kluyveromyces marxianus* called KMPROTEO 1.

5. A method for Fed-batch fermenting the strain *Kluyveromyces marxianus* called KMPROTEO 1, described in claim 2, includes the formulation of the medium "C", optimised for single-cell protein production at any scale.

6. A product obtained as a result of the process, described in claim 2, consisting of a single-cell protein powder, which protein content is equal to or greater than 60%, is highly bioavailable and has a nitrogen content greater than or equal to 9.6%.

7. A powder product obtained as a result of the process, described in claim 2, which, as it is soluble, can be included in different food matrices, without changing the formulation or the profile of the product to which it is added.

8. A product obtained as a result of the process, described in claim 2, is a fine powder with a particle size of less than 250 microns.

9. The product obtained directly from the process, according to claim 6, is a water-soluble ingredient, which can be included in different food matrices, without changing the formulation or the nutritional profile of the product to which it is added, improving its organoleptic characteristics.
